# EUROPÄISCHE PATENTSCHRIFT

(11) **EP 3 108 908 B1**
(45) Veröffentlichungstag und Bekanntmachung des Hinweises auf die Patenterteilung: **03.11.2021**
(21) Anmeldenummer: 16172002.4
(22) Anmeldetag: 30.05.2016
(51) Int. Cl.: A61M 1/02, A61M 5/168

(54) **VORRICHTUNG ZUR KONTROLLIERTEN BLUTENTHAME VON EINEM PATIENTEN/SPENDER SOWIE KIT MIT EINER DERARTIGEN VORRICHTUNG**
DEVICE FOR CONTROLLED PICKUP OF BLOOD FROM A PATIENT/DONOR AND KIT COMPRISING SUCH A DEVICE
DISPOSITIF DE PRELEVEMENT CONTROLE DE SANG SURE UN PATIENT/DONNEUR ET KIT COMPRENANT UN TEL DISPOSITIF

(30) Priorität: 24.06.2015 DE 202015103317 U
(43) Veröffentlichungstag der Anmeldung: 28.12.2016
(73) Patentinhaber: LMB Technologie GmbH, 85445 Schwaig (DE)
(72) Erfinder: Jentsch, Klaus D., 85445 Schwaig (DE)
(74) Vertreter: Winter, Brandl - Partnerschaft mbB

(56) Entgegenhaltungen:
- EP-A2- 1 428 541
- US-A- 5 112 019
- US-A1- 2013 106 609
- None

## Beschreibung

### Gebiet der Erfindung

Die vorliegende Erfindung betrifft eine Vorrichtung zur kontrollierten Entnahme von Blut von einem Patienten, sowie ein Kit mit einer derartigen Vorrichtung.

### Technischer Hintergrund

Bei der kontrollierten Abgabe von Fluiden (z.B. von Infusionen) oder der Aufnahme von Fluiden (z.B. Blutentnahme, Blutspende) von einem Patienten wird herkömmlicherweise eine Vielzahl von Vorrichtungen verwendet. Beispielsweise wird das verwendete Gefäß zur Fluidaufnahme bzw. -abgabe meist auf einem höhenverstellbaren Tisch gelagert und die Fluidabgabe bzw. -aufnahme aus bzw. in das Gefäß wird über eine separate Bedieneinheit gesteuert. Die Verwendung separater Vorrichtungen ist jedoch zeitaufwendig und fehleranfällig, denn bevor mit der Abgabe bzw. Aufnahme von Fluiden begonnen werden kann, müssen zuerst die einzelnen Vorrichtungen wie z.B. der höhenverstellbare Tisch, die Bedieneinheit, eine Anzeigeeinheit für das Anzeigen des Betriebsverlaufs etc. miteinander verbunden werden und deren Einstellungen aufeinander abgestellt werden. Besonders im hektischen Klinikalltag kann es hierbei zu fehlerhaften Verbindungen zwischen einzelnen Vorrichtungen kommen oder die Höhe des höhenverstellbaren Tischs kann fehlerhaft eingestellt werden. Bei der Abgabe und Aufnahmen von Fluiden von einem Patienten ist jedoch die korrekte relative Höhenpositionierung zwischen dem Gefäß zur Fluidaufnahme bzw. -abgabe und dem Patienten für den Erfolg entscheidend.

Zum besseren Verständnis der vorliegenden Erfindung sei auf die US 5 112 019 A, in der eine Vorrichtung zur kontrollierten Abgabe und Aufnahme von Fluiden von einem Patienten - dort als "Motorized VB Pole Assembly" bezeichnet - beschrieben ist, auf die EP 1 428 542 A2, in der eine Stütze für einen Infusionsfluidbehälter - dort als "Infusion Fluid Container Support" bezeichnet - für den Einsatz in der Ophthalmologie beschrieben ist, und die US 2013/106609 A1, die Anordnungen bzw. Vorrichtungen zur Stütze eines Dialysegeräts - dort "Dialysis Machine Support Assemblies" bezeichnet - offenbart, verwiesen.

Der vorliegenden Erfindung liegt die Aufgabe zugrunde, eine Vorrichtung zur kontrollierten Blutentnahme von einem Patienten zu schaffen, mit der Fehler bei der Entnahme aufgrund eines fehlerhaften Aufbaus der Vorrichtung vermieden werden und bei der zudem die relative Höhenpositionierung zwischen dem Patienten und dem zur Blutentnahme verwendeten Gefäß zuverlässig eingestellt werden kann.

Diese Aufgabe wird gelöst durch die Vorrichtung mit den Merkmalen gemäß Anspruch 1 sowie durch ein Kit gemäß Anspruch 9 mit einer derartigen Vorrichtung.

### Kurzbeschreibung der Erfindung

Eine erfindungsgemäße Vorrichtung zur kontrollierten Entnahme von Blut von einem Patienten weist mindestens eine Anzeigeeinheit, mindestens eine Bedieneinheit, sowie mindestens einen Halteabschnitt für ein Blutaufnahmegefäß auf. Hierbei ist die Anordnung des Halteabschnitts an der Vorrichtung über eine mittels der Bedieneinheit eingegebene Benutzereingabe einstellbar. Da die verschiedenen Komponenten Anzeigeeinheit, Bedieneinheit und Halteabschnitt bereits in einer Vorrichtung integriert sind, entfällt durch diese Vorrichtung die Notwendigkeit, mehrere Einzelkomponenten miteinander vor der Anwendung zu kombinieren und zu verbinden.

Gemäß einem vorteilhaften und gegebenenfalls separat zu beanspruchenden Aspekt der Erfindung ist die relative Höhe des Halteabschnitts über einem Boden der Vorrichtung einstellbar. In anderen Worten ist erfindungsgemäß ein höhenverstellbarer Tisch oder ähnliches fest in der Vorrichtung zur kontrollierten Blutentnahme von einem Patienten integriert.

Gemäß einem weiteren vorteilhaften und gegebenenfalls separat zu beanspruchenden Aspekt der Erfindung ist die Anordnung des Halteabschnitts in Antwort auf eine über die Bedieneinheit eingegebene Benutzereingabe, vorzugsweise in Antwort auf die Auswahl eines Betriebsmodus der Vorrichtung, falls die erfindungsgemäße Vorrichtung Teil einer allgemeineren Vorrichtung zur kontrollierten Abgabe und Aufnahme von Fluiden ist, automatisch einstellbar ist. Hierbei sind vorzugsweise in der allgemeineren Vorrichtung im Vorhinein mehrere Betriebsmodi bzw. Funktionen der Vorrichtung und diesen jeweils fest zugeordnete Höheneinstellungen des Halteabschnitts gespeichert, welche in Antwort auf eine Benutzereingabe abgerufen und zur Einstellung der Höhe des Halteabschnitts verwendet werden. Beispielsweise wird in der Bedieneinheit die Funktion "Blutentnahme" (vorliegende Erfindung) von einem Benutzer ausgewählt. Bei der Blutentnahme ist es vorteilhaft, wenn zwischen dem liegendem Blutspender und dem Blutaufnahmegefäß bzw. Blutbeutel ein Höhenunterschied von ca. 40 cm besteht. Ist beispielsweise bekannt, dass bei der Blutentnahme der Patient meist in einer Höhe von ca. 70 cm über dem Boden liegt, so kann in der Vorrichtung der Funktion "Blutentnahme" die Höheneinstellung "30 cm" zugeordnet und diese Zuordnung in der Vorrichtung gespeichert sein. Wählt der Benutzer die Funktion "Blutentnahme" aus, so liest die erfindungsgemäße Vorrichtung die gespeicherte Zuordnung zwischen dieser Funktion und der Höheneinstellung "30 cm" aus und stellt die Höhe des Halteabschnitts automatisch und ohne eine weitere Benutzereingabe auf 30 cm über dem Boden ein.

Gemäß einem weiteren vorteilhaften und gegebenenfalls separat zu beanspruchenden Aspekt der Erfindung weist eine erfindungsgemäße Vorrichtung mindestens eine Leseeinheit zur Datenerfassung auf. Die Leseeinheit, z.B. ein Barcodeleser, kann hierbei ein beweglich mit der Vorrichtung verbundenes Handheld-Gerätsein oder baulich in der Vorrichtung integriert sein. Mit der mindestens einen Leseeinheit können beispielsweise Identifikationsdaten eines Gefäßes zur Blutentnahme von einem Patienten wie z.B. eines Blutbeutels eingelesen werden. Alternativ oder zusätzlich dazu können auch Identifikationsdaten beispielsweise von einem Armband eines Patienten eingelesen werden.

Ist die Leseeinheit in der erfindungsgemäßen Vorrichtung baulich integriert, bietet dies den Vorteil, dass die Leseeinheit nicht wie bei einem Handheld-Gerät zum Auslesen von Daten händisch ergriffenwerden muss. Stattdessen kann beispielsweise das Gefäß zur Blutentnahme, z.B. ein Blutbeutel, zum Auslesen der Daten über die Leseeinheit bewegt werden. Zu diesem Zweck ist eine etwaige zwischen der Leseeinheit und der Außenseite der Vorrichtung angeordnete Außenwand der Vorrichtung zumindest abschnittsweise als ein Sichtfenster ausgestaltet, durch welches die Leseeinheit die Daten erfassen kann.

Gemäß einem weiteren vorteilhaften und gegebenenfalls separat zu beanspruchenden Aspekt der Erfindung weist eine erfindungsgemäße Vorrichtung eine Warnanzeige, vorzugsweise in Form eines Lichtsignals auf, welche den Betriebsmodus und / oder eine Fehlfunktion der Vorrichtung anzeigt. Die Warnanzeige kann in Form eines ausgesandten Lichtstrahls (Lichtantenne), einer Warnleuchte oder auch als akustisches Signal ausgestaltet sein. Bei einer bevorzugten Ausführungsform der Erfindung ist die Warnanzeige als Lichtantenne ausgebildet. Die Farbe der Lichtantenne ist beispielsweise grün, wenn die Vorrichtung die Funktion "Blutentnahme" durchführt und blau, wenn die Blutentnahme beendet ist. Auch kann die Lichtantenne mit einer bestimmten Frequenz blinken, um beispielsweise eine fehlerhafte Funktion der Vorrichtung anzuzeigen. Verschiedene Frequenzen können hierbei verschiedene Fehler in verschiedenen Betriebsmodi der Vorrichtung anzeigen.

Gemäß einem weiteren vorteilhaften und gegebenenfalls separat zu beanspruchenden Aspekt der Erfindung weist eine erfindungsgemäße Vorrichtung zudem ein Schlauchschweißgerät auf, mit dem an die Vorrichtung angeschlossene Schläuche gleichzeitig geschnitten und versiegelt werden können.

Gemäß einem anderen vorteilhaften und gegebenenfalls separat zu beanspruchenden Aspekt der Erfindung weist eine erfindungsgemäße Vorrichtung zudem eine Datenschnittstelle auf, mit der von der mindestens einen Leseeinheit eingelesene Daten beispielsweise an eine Datenbank transferiert werden können oder mit in der Datenbank abgelegten Datensätzen abgeglichen werden können.

Um die Anwendung der Vorrichtung weiter zu vereinfachen wird ein mobiles Kit zur kontrollierten Blutentnahme von einem Patienten mit einer erfindungsgemäßen Vorrichtung bereitgestellt.

Ein erfindungsgemäßes Kit umfasst vorzugsweise einen Koffer, welcher eine erfindungsgemäße Vorrichtung zur kontrollierten Entnahme von Blut enthält. Hierbei können Funktionen der erfindungsgemäßen Vorrichtung in den Wänden des Gehäuses bzw. des Koffers des Kits integriert sein: Beispielsweise kann der aufgeklappte Deckel des Gehäuses als Führungsschiene dienen, an welcher der Halteabschnitt in seiner Höhenpositionierung verschiebbar ist. Hierbei ist der aufgeklappte Deckel des Gehäuses im Wesentlichen rechtwinklig zu dem Körper und Boden des Gehäuses angeordnet.

Gemäß einem vorteilhaften und gegebenenfalls separat zu beanspruchenden Aspekt der Erfindung ist das mobile Kit von einem Menschen, beispielsweise von einer Krankenschwester, tragbar.

Durch das erfindungsgemäße Kit wird die Durchführung einer kontrollierten Entnahme von Blut einem Patienten wesentlich vereinfacht und benutzerfreundlicher, denn die verschiedenen Komponenten der Vorrichtung zur kontrollierten Entnahme von Blut einem Patienten sind in dem Kit bereits korrekt miteinander verbunden und müssen daher nicht vor der Anwendung miteinander verbunden werden. Zudem erlaubt das Kit den einfachen Transport der erfindungsgemäßen Vorrichtung.

Gemäß einem vorteilhaften und gegebenenfalls separat zu beanspruchenden Aspekt der Erfindung ist das Gehäuse des Kits, beispielsweise des Koffers, derart ausgestaltet, dass an dem Gehäuse mindestens ein zusätzliches Modul anbringbar ist. Dieses zusätzliche Modul kann beispielsweise eine Ablagefläche, ein Tablett oder eine Aufnahme sein.

Um die Mobilität des erfindungsgemäßen Kits weiter zu verbessern, ist das Kit gemäß einem weiteren Aspekt der Erfindung mit einer Batterie ausgestattet.

### Figurenbeschreibung

Weitere Merkmale und Vorteile der vorliegenden Erfindung ergeben sich aus der folgenden Beschreibung eines Ausführungsbeispiels unter Bezugnahme auf die zugehörigen Figuren. Hierbei zeigt:
Fig. 1 eine Perspektivansicht eines mobilen Kits zur kontrollierten Abgabe und Aufnahme von Fluiden von einem Patienten mit einer erfindungsgemäßen Vorrichtung zur kontrollierten Entnahme von Blut von dem Patienten,
Fig. 2 eine Frontansicht des Kits aus Fig. 1,
Fig. 3 eine Seitenansicht des Kits aus Fig. 1, und
Fig. 4 eine Draufsicht des Kits aus Fig. 4.

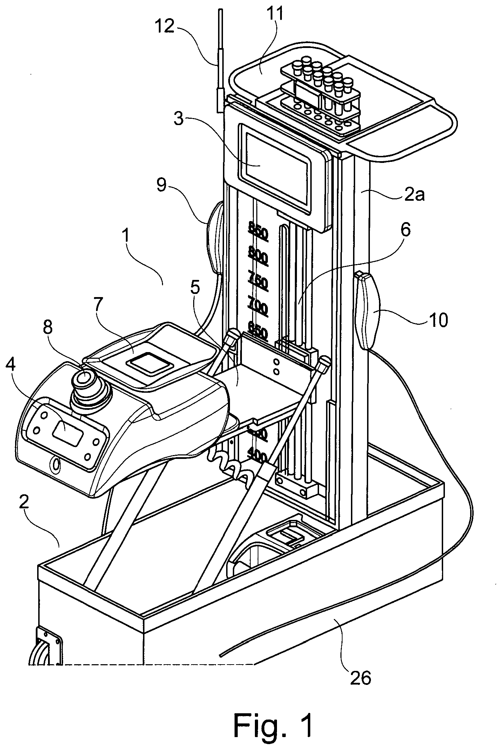

Fig. 1 zeigt ein mobiles Kit 1 mit einem Gehäuse 2, in welchem eine Vorrichtung mit einer Anzeigeeinheit 3, einer Bedieneinheit 4, und einem Halteabschnitt 5 für ein hier nicht gezeigtes Fluid-Aufnahme/-Abgabegefäß, wie beispielsweise einen Blutbeutel, angeordnet sind. Von der nachfolgenden Vorrichtung betrifft die vorliegende Erfindung jedoch nur den Teil der Blutennahme. Das Gehäuse 2 besteht aus einem Gehäusedeckel 2a und einem Gehäusekörper 2b. In der in Fig. 1 gezeigten Ansicht ist der Gehäusedeckel 2a geöffnet und im Wesentlichen rechtwinklig zu der Oberkante bzw. dem Boden des Gehäusekörpers 2b des Kits angeordnet. Entlang des Gehäusedeckels 2a ist eine Führungsschiene 6 ausgebildet, entlang der der Halteabschnitt 5 höhenverstellbar gelagert ist. Hierbei zeigt eine Skale die Höhenposition des Halteabschnitts 5 an. Auf dem Halteabschnitt 5 ist in dieser Ausführungsform eine Mischwaage 7 angeordnet, auf welcher ein Fluidaufnahme/-abgabegefäß, wie beispielsweise einen Blutbeutel, aufgelegt werden kann. Durch die Messung des Gewichts des Fluid-Aufnahme/- Abgabegefäßes kann der Füllstand des Fluids in dem Fluidaufnahme/-abgabegefäß überwacht werden. Mittels einer steuerbaren Schlauchklemme 8 kann der Zu- bzw. Abfluss von Fluid von dem Fluidaufnahme/-abgabegefäß gesteuert werden. Mit der Bedieneinheit 4 kann ein Benutzer den Betrieb der in dem Gehäuse 2 des Kits 1 enthaltenen Vorrichtung steuern. Beispielsweise kann der Benutzer verschiedenen Funktionen über die Bedieneinheit 4 auswählen. Auf der Anzeigeeinheit 3 werden verschiedenen Information bezüglich des Betriebs der Vorrichtung angezeigt, wie beispielsweise die gewählte Funktion, die Identifikationsdaten eines Patienten oder eines Fluid-Aufnahme/- Abnahmegefäßes oder die Zeitdauer, für die die Vorrichtung bereits diese Funktion durchführt. Die Anzeigeeinheit 3 ist für einen Benutzer sehr einfach einsehbar an dem bei geöffnetem Gehäuse dem Gehäusekörper 2b abgewandten Ende des Gehäusedeckels 2a angeordnet. Weiterhin weist das Kit 1 eine Leseeinheit 9 auf, welche hier ein Barcodeleser ist. Mit diesem Barcodeleser 9 lassen sich beispielsweise Identifikationsdaten eines Patienten oder eines Fluid-Aufnahme/-Abnahmegefäßes erfassen. Zusätzlich weist das Kit 1 ein Schlauchschweißgerät 10 auf, mit welchem sich Fluidleitungen und sonstige Schläuche gleichzeitig schneiden und versiegeln lassen. Weiterhin ist an dem bei geöffnetem Gehäuse dem Gehäusekörper 2b abgewandten Ende des Gehäusedeckels 2a ein zusätzliches Modul angeordnet. In dieser Ausführungsform ist das zusätzliche Modul ein Tablett 11, welches auf den Gehäusedeckel 2a aufgesteckt ist. Der Betrieb der Vorrichtung kann über eine Warnanzeige in Form einer Lichtantenne 12 überwacht werden. Diese Lichtantenne zeigt durch Änderungen der Farbe und / oder der Blinkfrequenz den Betriebsmodus der Vorrichtung sowie eine etwaige Fehlfunktion der Vorrichtung an.

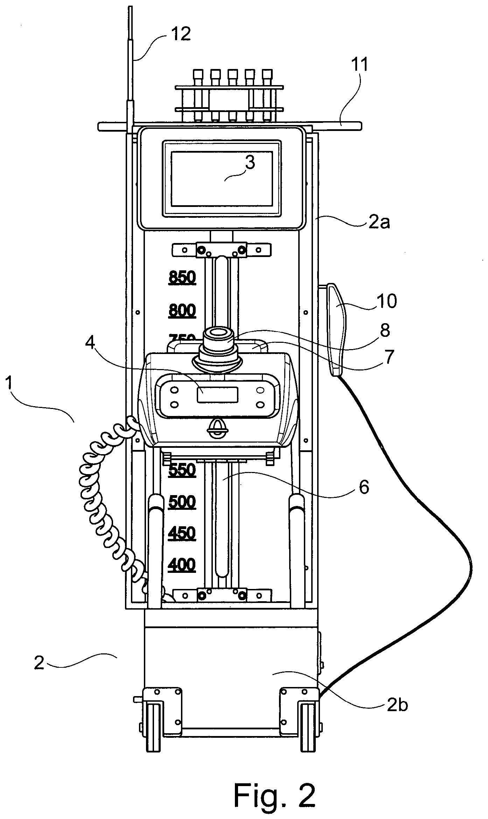

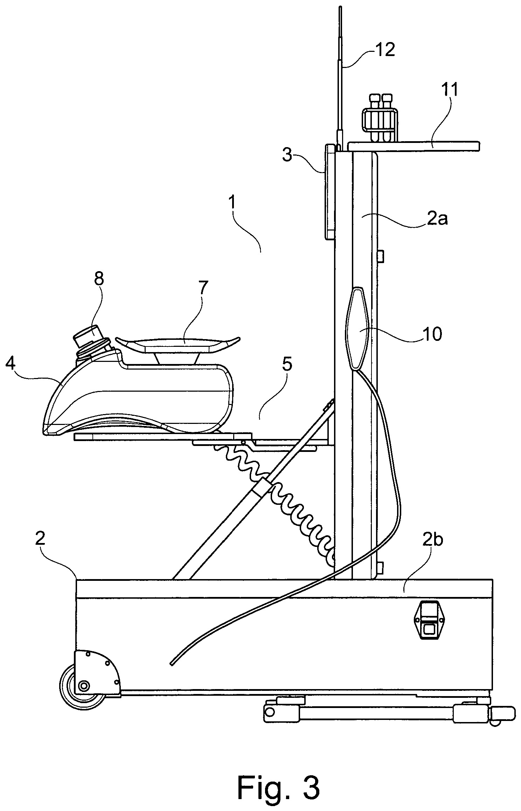

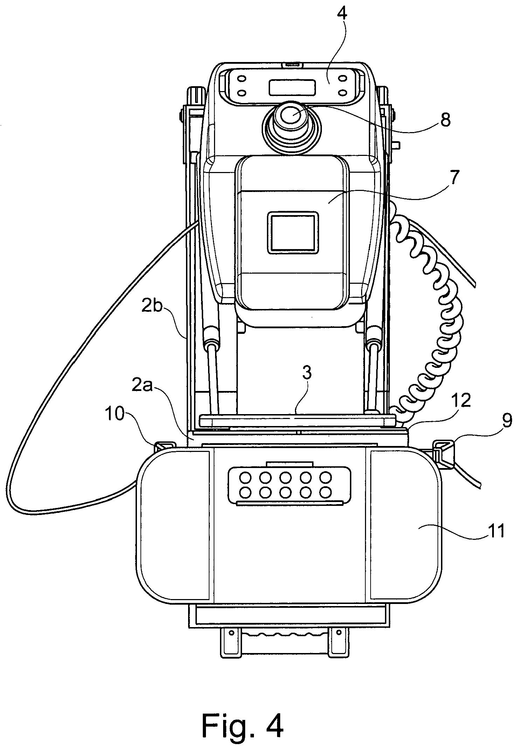

Die Figuren 2 bis 4 zeigen dieselbe Vorrichtung wie die Fig. 1. Gleiche oder ähnliche Bauteile sind hierbei mit den gleichen Bezugszeichen bezeichnet wie in Fig. 1.

## Patentansprüche

1. Vorrichtung zur kontrollierten Blutentnahme von einem Patienten, mit:
- mindestens einer Anzeigeeinheit (3),
- mindestens einer Bedieneinheit (4), sowie
- mindestens einem Halteabschnitt (5) für ein Blutentnahmegefäß, wobei die Anordnung des Halteabschnitts (5) an der Vorrichtung über eine mittels der Bedieneinheit (4) eingegebene Benutzereingabe einstellbar ist.

2. Vorrichtung nach Anspruch 1, **dadurch gekennzeichnet, dass** die relative Höhe des Halteabschnitts (5) über einem Boden der Vorrichtung einstellbar ist.

3. Vorrichtung nach einem der vorhergehenden Ansprüche, **dadurch gekennzeichnet, dass** die Anordnung des Halteabschnitts (5) in Antwort auf eine über die Bedieneinheit (4) eingegebene Benutzereingabe, vorzugsweise in Antwort auf die Auswahl eines Betriebsmodus der Vorrichtung, automatisch einstellbar ist.

4. Vorrichtung nach einem der vorhergehenden Ansprüche, **gekennzeichnet durch** mindestens eine Leseeinheit (9) zur Datenerfassung.

5. Vorrichtung nach Anspruch 4, **dadurch gekennzeichnet, dass** die Leseeinheit (9) ein beweglich mit der Vorrichtung verbundenes Handheld-Gerät ist oder baulich in der Vorrichtung integriert ist.

6. Vorrichtung nach einem der vorhergehenden Ansprüche **gekennzeichnet durch** eine Warnanzeige (12), vorzugsweise in Form eines Lichtsignals, welche den Betriebsmodus und / oder eine Fehlfunktion der Vorrichtung anzeigt.

7. Vorrichtung nach einem der vorhergehenden Ansprüche **gekennzeichnet durch** weiterhin ein Schlauchschweißgerät (10), mit dem an die Vorrichtung angeschlossene Schläuche gleichzeitig geschnitten und versiegelt werden können.

8. Vorrichtung nach einem der vorhergehenden Ansprüche, **gekennzeichnet durch** weiterhin eine Datenschnittstelle, vorzugsweise zum Datenaustausch mit einer Datenbank.

9. Mobiles Kit (1) zur kontrollierten Blutentnahme von einem Patienten mit einer Vorrichtung nach einem der Ansprüche 1 bis 8.

10. Mobiles Kit (1) nach Anspruch 9, **dadurch gekennzeichnet, dass** das Kit (1) tragbar ist.

11. Mobiles Kit (1) nach Anspruch 9 oder 10, **dadurch gekennzeichnet, dass** das Kit (1) ein Gehäuse (2), vorzugsweise in Form eines Koffers, aufweist.

12. Mobiles Kit (1) nach Anspruch 11, **dadurch gekennzeichnet, dass** das Gehäuse (2) derart ausgestaltet ist, dass an dem Gehäuse (2) mindestens ein zusätzliches Modul (11) anbringbar ist.

13. Mobiles Kit (1) nach Anspruch 12, **dadurch gekennzeichnet, dass** das mindestens eine zusätzliche Modul (11) eine Ablagefläche oder eine Aufnahme ist.

14. Mobiles Kit (1) nach einem der Ansprüche 9 bis 13, mit weiterhin einer Batterie.

## Claims

1. A device for controlled blood collection from a patient, comprising:
at least one display unit (3),
at least one operating unit (4), and
at least one holding section (5) for a blood collection vessel, wherein the arrangement of the holding section (5) on the device is adjustable via a user input entered using the operating unit (4).

2. The device according to claim 1, **characterized in that** the relative height of the holding section (5) above a bottom of the device is adjustable.

3. The device according to any one of the preceding claims, **characterized in that** the arrangement of the holding section (5) is automatically adjustable in response to a user input entered via the operating unit (4), preferably in response to the selection of an operating mode of the device.

4. The device according to any one of the preceding claims, **characterized by** at least one reading unit (9) for data acquisition.

5. The device according to claim 4, **characterized in that** the reading unit (9) is a handheld device, which is movably connected to the device, or is structurally integrated into the device.

6. The device according to any one of the preceding claims **characterized by** a warning indicator (12), preferably in the form of a light signal, which indicates the operating mode and/or a malfunction of the device.

7. The device according to any one of the preceding claims further **characterized by** a tube welding device (10) with which tubes connected to the device can be cut and sealed at the same time.

8. The device according to any one of the preceding claims, further **characterized by** a data interface, preferably for data exchange with a database.

9. A mobile kit (1) for controlled blood collection from a patient comprising a device according to any one of claims 1 to 8.

10. The mobile kit (1) according to claim 9, **characterized in that** the kit (1) is portable.

11. The mobile kit (1) according to claim 9 or 10, **characterized in that** the kit (1) comprises a housing (2), preferably in the form of a suitcase.

12. The mobile kit (1) according to claim 11, **characterized in that** the housing (2) is designed such that at least one additional module (11) is attachable to the housing (2).

13. The mobile kit (1) according to claim 12, **characterized in that** the at least one additional module (11) is a shelf or a receptacle.

14. The mobile kit (1) according to any one of claims 9 to 13, further comprising a battery.

## Revendications

1. Dispositif pour le prélèvement sanguin contrôlé d'un patient, avec :
- au moins une unité d'affichage (3),
- au moins une unité de commande utilisateur (4), ainsi que
- au moins une partie de retenue (5) pour un contenant de prélèvement sanguin, dans lequel la disposition de la partie de retenue (5) sur le dispositif peut être réglée par l'intermédiaire d'une entrée d'utilisateur entrée au moyen de l'unité de commande utilisateur (4).

2. Dispositif selon la revendication 1, **caractérisé en ce que** la hauteur relative de la partie de retenue (5) au-dessus d'un fond du dispositif est réglable.

3. Dispositif selon l'une quelconque des revendications précédentes, **caractérisé en ce que** la disposition de la partie de retenue (5) en réponse à une entrée d'utilisateur entrée par l'intermédiaire de l'unité de commande utilisateur (4), de préférence en réponse à la sélection d'un mode de fonctionnement du dispositif, est réglable automatiquement.

4. Dispositif selon l'une quelconque des revendications précédentes, **caractérisé par** au moins une unité de lecture (9) pour la détection de données.

5. Dispositif selon la revendication 4, **caractérisé en ce que** l'unité de lecture (9) est un appareil portatif relié au dispositif de manière mobile ou est intégrée dans le dispositif par construction.

6. Dispositif selon l'une quelconque des revendications précédentes, **caractérisé par** un indicateur de chaleur (12), de préférence sous forme d'un signal lumineux, lequel indique le mode de fonctionnement et/ou un dysfonctionnement du dispositif.

7. Dispositif selon l'une quelconque des revendications précédentes, **caractérisé en outre par** un appareil de soudage de tubes (10), avec lequel des tubes raccordés au dispositif peuvent être coupés et scellés simultanément.

8. Dispositif selon l'une quelconque des revendications précédentes, **caractérisé en outre par** une interface de données, de préférence pour l'échange de données avec une banque de données.

9. Kit mobile (1) pour le prélèvement sanguin contrôlé d'un patient avec un dispositif selon l'une quelconque des revendications 1 à 8.

10. Kit mobile (1) selon la revendication 9, **caractérisé en ce que** le kit (1) est portatif.

11. Kit mobile (1) selon la revendication 9 ou 10, **caractérisé en ce que** le kit (1) présente un boîtier (2), de préférence sous forme d'une malle.

12. Kit mobile (1) selon la revendication 11, **caractérisé en ce que** le boîtier (2) est conçu de telle sorte qu'au moins un module (11) additionnel peut être monté sur le boîtier (2).

13. Kit mobile (1) selon la revendication 12, **caractérisé en ce que** l'au moins un module (11) additionnel est une surface de réception ou un logement.

14. Kit mobile (1) selon l'une quelconque des revendications 9 à 13, avec en outre une batterie.
